# EUROPEAN PATENT SPECIFICATION

(11) **EP 1 774 334 B1**
(45) Date of publication and mention of the grant of the patent: **04.10.2017**
(21) Application number: 05776764.2
(22) Date of filing: 01.08.2005
(51) Int. Cl.: G01N 33/68, G01N 33/543, C12Q 1/68, B01D 15/36, B01D 15/38, B01J 20/28, B01J 20/32, C12N 15/10

(54) **USE OF MAGNETIC MATERIAL TO DIRECT ISOLATION OF COMPOUNDS AND FRACTIONATION OF MULTIPART SAMPLES**
VERWENDUNG VON MAGNETISCHEM MATERIAL ZUR DIREKTEN ISOLIERUNG VON SUBSTANZEN UND FRAKTIONIERUNG VON PROBEN, DIE MEHRERE KOMPONENTEN ENTHALTEN
UTILISATION D'UN MATERIAU MAGNETIQUE PERMETTANT DE PROCEDER A L'ISOLEMENT DE COMPOSES ET LE FRACTIONNEMENT D'ECHANTILLONS CONSTITUES DE PLUSIEURS PARTIES

(30) Priority: 03.08.2004 US 598118 P
(43) Date of publication of application: 18.04.2007
(73) Proprietor: Becton, Dickinson and Company, Franklin Lakes, NJ 07417-1880 (US)
(72) Inventor: FORT, Thomas, L., Finksburg, MD 21048 (US); COLLIS, Matthew, P., Seven Valleys, PA 17360 (US); GENTLE, Jr., Thomas, M., Red Lion, PA 1736056 (US)
(74) Representative: dompatent von Kreisler Selting Werner - Partnerschaft von Patent- und Rechtsanwälten mbB
(86) International application number: PCT/US2005/027209
(87) International publication number: WO 2006/017428

(56) References cited:
- EP-A- 0 919 285
- EP-A- 1 589 105
- EP-A1- 0 016 552
- WO-A-94/11103
- WO-A-99/29703
- WO-A-2006/017427
- WO-A-2006/020280
- US-A- 6 008 002
- US-A- 6 126 835
- US-B1- 6 534 262
- VILLANUEVA J ET AL: "SERUM PEPTIDE PROFILING BY MAGNETIC PARTICLE-ASSISTED, AUTOMATED SAMPLE PROCESSING AND MALDI-TOF MASS SPECTROMETRY", ANALYTICAL CHEMISTRY, AMERICAN CHEMICAL SOCIETY, US, vol. 76, no. 6, 15 March 2004 (2004-03-15) , pages 1560-1570, XP001196643, ISSN: 0003-2700, DOI: 10.1021/AC0352171
- GOVORUKHINA N I ET AL: "Sample preparation of human serum for the analysis of tumor markers - Comparison of different approaches for albumin and gamma-globulin depletion", JOURNAL OF CHROMATOGRAPHY, ELSEVIER SCIENCE PUBLISHERS B.V, NL, vol. 1009, no. 1-2, 15 August 2003 (2003-08-15), pages 171-178, XP004447859, ISSN: 0021-9673, DOI: 10.1016/S0021-9673(03)00921-X
- STEELE L F ET AL: "EFFICIENT AND SPECIFIC REMOVAL OF ALBUMIN FROM HUMAN SERUM SAMPLES", MOLECULAR & CELLULAR PROTEOMICS, AMERICAN SOCIETY FOR BIOCHEMISTRY AND MOLECULAR BIOLOGY, US, vol. 2, no. 4, 1 January 2003 (2003-01-01) , pages 262-270, XP001160903, ISSN: 1535-9476

## Description

### Technical Field

The present invention relates to a method of depleting high abundance proteins from a serum sample prior to evaluating said sample by an analytical instrument, specifically using paramagnetic partices to which protein A and/or native protein G is bound.

### Background Art

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Historically, purification schemes have been predicated on differences in the molecular properties of size, charge and solubility between the compound to be purified and the undesired contaminants contained therein. Protocols based on these parameters include size exclusion chromatography, ion exchange chromatography, differential precipitation and the like.

Size exclusion chromatography, otherwise known as gel filtration or gel permeation chromatography, relies on the penetration of molecules in a mobile phase into the pores of stationary phase particles. Differential penetration is a function of the hydrodynamic volume of the particles. Accordingly, under ideal conditions, the larger molecules are excluded from the interior of the particles, while the smaller molecules are accessible to this volume, and the order of elution can be predicted by the size of the compound because a linear relationship exists between elution volume and the log of the molecular weight.

Ion exchange chromatography involves the interaction of charged functional groups in the sample with ionic functional groups of opposite charge on an adsorbent surface. Two general types of interaction are known. The first is anionic exchange chromatography, which is mediated by negatively charged functional groups interacting with positively charged surfaces. The second is cationic exchange chromatography, which is mediated by positively charged functional groups interacting with negatively charged surfaces.

More recently, affinity chromatography and hydrophobic interaction chromatography techniques have been developed to supplement the more traditional size exclusion and ion exchange chromatographic protocols. Affinity chromatography relies on the interaction of the compound with an immobilized ligand. The ligand can be specific for the particular compound of interest, in which case the ligand is a substrate, substrate analog, inhibitor or antibody. Alternatively, the ligand may be able to react with a number of compounds. Such general ligands as adenosine monophosphate, adenosine diphosphate, nicotine adenine dinucleotide or certain dyes may be employed to recover a particular class of proteins.

Metal affinity partitioning exploits the affinity of transition metal ions for electron-rich amino acid residues, such as histidine and cysteine, accessible on the surfaces of some proteins. When the metal ion is partially chelated and coupled to a linear polymer, such as polyethylene glycol ("PEG"), the resulting polymer-bound metal chelate can be used to enhance the partitioning of metal binding proteins into the polymer-rich phase of a PEG-salt or PEG-dextran aqueous two-phase system.

The application of a metal affinity ligand for the isolation of proteins is known. It has been demonstrated that histidine- and cysteine-containing proteins could be chromatographically separated from each other using a support that had been functionalized with a chelator, such as iminodiacetic acid ("IDA"), which is attached to a polymer spacer and bound to a metal such as copper, zinc or nickel. Immobilized metal affinity chromatography ("IMAC") has evolved into a useful tool for protein chromatography and a number of IDA-based IMAC resins are now commercially available.

Many problems occur when using metal chelates to purify a target protein from a crude preparation. One problem in particular centers on the selectivity of the ligand for the target protein, i.e., the ligand can be under or over selective in binding the target protein. There also is a problem of nitrogen-containing compounds in a crude system inhibiting ligand binding to the target protein. Finally, there is a problem relating to protein solubility and potential precipitation of proteins by the salt used in an aqueous, two-phase partitioning system. All of these problems can dramatically affect the target protein yield.

U.S. Patent No. 5,907,035 (Guinn) attempts to address the problems associated with metal chelation by using an aqueous, two-phase metal affinity partitioning system for purifying target proteins from crude protein solutions. The method includes the use of salts and inert hydrophobic molecules, such as polymers, to produce the aqueous two-phase system and the use of a polymer-chelator-metal complex to purify target proteins by selectively binding them to the complex.
Govorukhina N I et al. (Journal of Chromatography, 2003, Vol. 1009, no. 1-2, pages 171-178) recognized that methods for detection of proteins and peptides (lower abundant proteins) in biological fluids can be masked by the presence of highly abundant proteins. Several ways to reduce levels of albumin and γ-globulins in human serum based on specific antibodies, dye ligands (for albumin) and protein A or G (for γ-globulines) were tested. The depleted serum was analyzed for the presence of the abundant proteins as well as for other serum proteins that should remain after depletion. However, the methods tested do not make use of paramagnetic particles.
Steele L F et al. (Molecular and Cellular Proteomics, American Society for Biochemisty and Molecular Biology, 2003, Vol. 2, No. 4, pages 262-270) also recognized that the high abundance of albumin and immunoglobulins in serum can interfere with the resolution and sensitivity of proteome profiling techniques. The authors used monoclonal antibodies against human serum albumin (HAS) in order to develop an immunoaffinity resin to remove HSA present in serum. They also used protein G resin in conjunction with the immunoaffinity resin they developed to remove Ig proteins and HAS in a single step. Thus, the methods utilized also do not make use of paramagnetic particles.
EP 0 016 552 A1 relates to the fractionation of cells to isolate a homogeneous population of a specific cell type or magnetically sorting cells, bacteria or viruses. To accomplish the sorting, magnetic microspheres coated with staphylococcal protein A and antibodies was used. However, there is no discussion or suggestion as to how magnetic coated particles with protein A and antibodies can be used to deplete highly abundant proteins, such as albumin and immunoglobulins from serum samples prior to analysis.

An effective and automated method of rapidly isolating small molecule compounds, macromolecules, or protein from crude samples has not been available. Precipitation techniques are still crude and difficult to automate. Chromatography is expensive and time consuming. Thus, there remains a need for a technique to rapidly fractionate and isolate compounds in crude chemically diverse samples.

### Disclosure of Invention

It is an object of the present invention to provide a capture technology that can be utilized in both an ion-exchange or an affinity-based method.

It is a further object of the present invention to provide a robust and inexpensive means to fractionate an organic or biological sample containing a mixture of compounds.

It is yet another object of the present invention to provide methods for separating compounds from samples by using ligands or functional groups with a specific affinity for target compounds.

It is another object of the present invention to use electronic charge differences between compounds and charged functional groups or ligands attached to paramagnetic particles to separate and isolate compounds. The present invention concerns a method for depleting high abundance proteins from a serum sample prior to evaluating the sample by an analytical instrument, said sample comprising a target compound and one or more compounds not of interest, the method comprising:
a) adding at least one paramagnetic particle to a sample receptacle containing a serum sample comprising the target compound and compounds not of interest, wherein said paramagnetic particle has one or more ligands covalently attached thereto and said one or more ligands have an affinity for one or more of the compounds not of interest in said sample, wherein said one or more ligands are selected from the group consisting of protein A and native protein G; wherein
   when the ligand is native protein G the one or more compounds not of interest are selected from immunoglobulins and albumin, and
   when the ligand is protein A the one or more compounds not of interest are selected from immunoglobulins; and wherein
   said at least one paramagnetic particle is selected from the group consisting of: a metal selected from the group consisting of iron, cobalt and nickel; and a metallic compound chosen from the group consisting of iron oxide, iron sulfide, iron chloride, ferric hydroxide, and ferrosoferric oxide;
b) contacting the ligands with the one or more compounds not of interest to form a particlecompound complex;
c) immobilizing the complex by applying an external magnetic field; and
d) removing from the receptacle the portion of the sample not immobilized by the magnetic field, wherein the sample thus removed contains the target compound.
In a further embodiment, the method of the present invention further comprises the step e) evaluating the removed portion of the sample by mass spectrometry.
Although much of the following disclosure does not explicitly concern the claimed invention, it nonetheless serves to provide valuable background information in respect of the field in general.

In order to provide a more effective and efficient technique for the purification and manipulation of compounds, the present disclosure describes compositions useful for binding a target compound. The target compound may be a small molecule compound, protein, peptide, or polynucleotide. The compositions include a paramagnetic particle, with a ligand or functional group attached, capable of binding a target compound based on the affinity of the ligand or functional group for the target compound. The disclosure also describes such a composition packaged as a kit, as well as methods utilizing such a composition.

This disclosure provides methods for isolating one or more compounds from a multipart sample. The methods may be initiated by adding at least one paramagnetic particle to a sample comprising one or more target compounds. The at least one paramagnetic particle has attached thereto one or more functional groups or ligands, with each functional group or ligand having an affinity for one or more of the target compounds in the sample. The methods continue by contacting the functional groups or ligands with the target compounds to form a complex. The complex is immobilized by an external magnetic field. The remaining portion of the sample not immobilized is removed leaving the target compounds for further processing. The complex can be further manipulated by removing the magnetic field thereby freeing the complex so that additional chemistry or purification methods can be performed on the complex.Alternatively, compounds other than the compound of interest could be bound in complex and separated from the remaining solution. The solution that is not immobilized could be separated from the bound material and manipulated as needed in this more concentrated state. A method of the disclosure but not according to the invention for isolating a target compound from a sample can
comprise: a) adding at least one paramagnetic particle chosen from the group consisting of iron oxide, iron sulfide, iron chloride, ferric hydroxide, and ferrosoferric oxide to a sample comprising one or more target compounds, wherein said at least one paramagnetic particle has one or more ligands or functional groups attached thereto, said ligands or functional groups having an affinity for one or more of the target compounds in said sample; b) contacting the one or more ligands or functional groups with the one or more target compounds to form a complex; c) immobilizing the complex by applying a magnetic field; d) removing the portion of the sample not immobilized by the magnetic field; e) removing the magnetic field to release the complex; f) eluting said target compounds from said complex; g) immobilizing said paramagnetic particles; and h) retrieving said target compounds.

A method of the present disclosure may be performed as set forth above, and wherein said one or more ligands or functional groups are covalently bound to the at least one paramagnetic particle.

A method of the present disclosure may be performed as set forth above, and wherein said one or more ligands or functional groups are specific for a target compound selected from the group consisting of an antibody, an antigen, a hapten, a receptor, an enzyme, a polypeptide, a protein and a polynucleotide.

A method of the present disclosure may be performed as set forth above, and wherein the at least one paramagnetic particle is a metal selected from the group consisting of iron, cobalt, and nickel.

A method of the present disclosure may be performed as set forth above, and wherein said one or more ligands or functional groups are attached through biological linkages.

A method of the present disclosure may be performed as set forth above, and wherein said biological linkages are selected from the group consisting of streptavidin-biotin, avidin-biotin, carbohydrates-lectins, and enzyme-enzyme inhibitors.

An alternative method for isolating a target compound from a sample comprising one or more target compounds and compounds not of interest, the method comprising: a) adding at least one paramagnetic particle to a sample comprising one or more target compounds, wherein said at least one paramagnetic particle has one or more ligands attached thereto, said one or more ligands having an affinity for one or more of target compounds not of interest in said sample; b) contacting the ligands with the compounds not of interest to form a complex; c) immobilizing the complex by applying a magnetic field; and d) removing the portion of the sample not immobilized by the magnetic field, wherein said sample thus removed contains the one or more target compounds.

In the method of the present invention as described above, said one or more ligands are covalently bound to the paramagnetic particle.

A method of the present disclosure may be performed as described above, and wherein said one or more ligands are specific for a compound selected from the group consisting of an antibody, an antigen, a hapten, a receptor, an enzyme, a polypeptide, a protein, and a polynucleotide. In the method of the present invention, said one or more ligands are as defined in claim 1 and have an affinity for the specific one or more compounds not of interest as defined in claim 1.
In the method of the present invention the at least one paramagnetic particle is selected from the group consisting of 1) a metal selected from the group consisting of iron, cobalt and nickel, and 2) a metallic compound selected from the group consisting of iron oxide, iron sulfide, iron chloride, ferric hydroxide and ferrosoferric oxide.

A method of the present disclosure may be performed as described above, and wherein said one or more ligands are attached through biological linkages.

A method of the present disclosure may be performed as described above, and wherein said biological linkages are selected from the group consisting of streptavidin-biotin, avidin-biotin, carbohydrates-lectins, and enzyme-enzyme inhibitors. A further alternative method (not according to the invention) for isolating compounds from a sample may comprise: a) adding at least one paramagnetic particle to a sample comprising one or more target compounds, wherein said at least one paramagnetic particle and said one or more target compounds have a charge difference; b) generating a complex between said one or more target compounds and said at least one paramagnetic particle; c) immobilizing the complex by applying a magnetic field; d) separating the complex immobilized by the magnetic field from the sample; e) removing the magnetic field to release the complex; f) eluting said target compounds from said complex; g) immobilizing said paramagnetic particles; and h) retrieving said target compounds.

A method of the disclosure may be performed as described above, and wherein the at least one paramagnetic particle has one or more charged functional groups attached thereto to provide the at least one paramagnetic particle with an overall charge.

A method of the present disclosure may be performed as described above, and further comprising the steps of centrifuging, filtration, purifying via affinity chromatography and resolving the complex prior to applying the magnetic field.

A method of the present disclosure may be performed as described above, and wherein the sample is altered by changing the sample pH.

A method of the present disclosure may be performed as described above, and wherein the sample is altered by changing the ionic strength.

A method of the present disclosure may be performed as described above, and wherein the at least one paramagnetic particle is a metal selected from the group consisting of iron, cobalt, and nickel.

The present disclosure also relates to kits for isolating proteins from samples, with the kits comprising a combination of some, or all, of the constituents described above.

Other objects, purposes and advantages of the present invention will become apparent with the following description of the invention.

### Brief Description of the Drawings

The foregoing and other features, aspects and advantages of the present invention will become apparent from the following description, appended claims and the exemplary embodiments shown in the drawing, which is briefly described below. It should be noted that, unless otherwise specified, like elements have the same reference numbers.

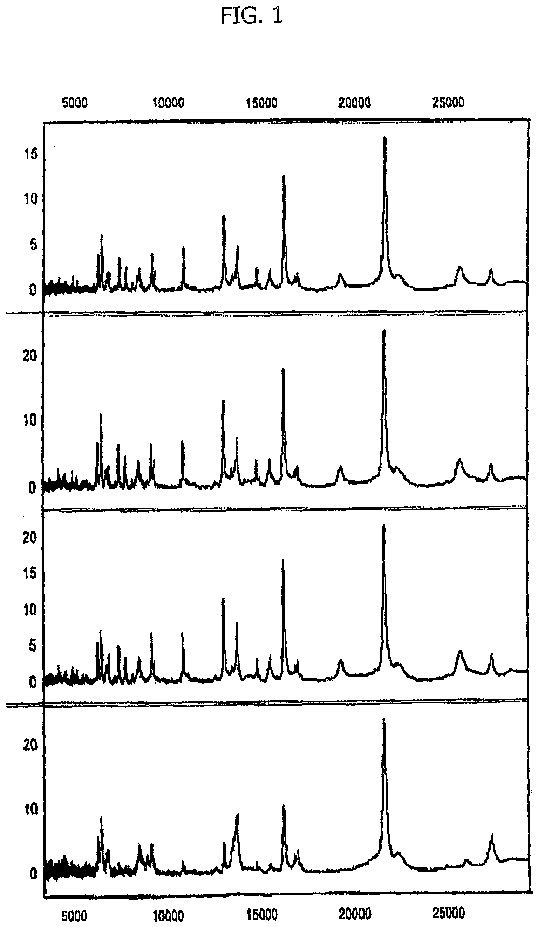

Fig. 1 is a side by side comparison of mass spectrometry chromatographs on human plasma samples illustrating the enhanced resolution gained by pre-treating the plasma samples with magnetic particles having one or more protein affinity ligands or functional groups attached.

### Modes for Carrying Out The Invention

The principles of the present invention will now be further described by the following discussion of certain illustrative embodiments thereof and by reference to the foregoing drawing figure.

This disclosure provides methods for isolating one or more compounds from a multipart sample. The methods may be initiated by adding at least one paramagnetic particle to a sample comprising one or more target compounds. The at least one paramagnetic particle has attached thereto one or more ligands or functional groups, with each ligand or functional group having an affinity for one or more of the target compounds in the sample. The methods continue by contacting the ligands or functional groups with the target compounds to form a complex. The complex is immobilized by an external magnetic field. The sample not immobilized is removed leaving the target compounds for further processing. The complex can be further manipulated by removing the magnetic field thereby freeing the complex so that additional chemistry or purification methods can be performed on the complex.

The present disclosure describes the use of at least one electronically charged paramagnetic particle to differentially bind and separate target compounds having a charge opposite that of the at least one paramagnetic particle. Electronic charge differences can be generated between the at least one paramagnetic particle and one or more of the target compounds by altering the sample.

As used herein, the term "paramagnetic particle(s)" means particle(s) capable of having a magnetic moment imparted to them when placed in a magnetic field. Typically, the paramagnetic particles consist of either metallic iron, cobalt or nickel. These elements are the only known to exist in a paramagnetic state while in their ground or zero oxidation state. In addition to these three metals, organic and organometallic compounds also possess paramagnetic properties. The metallic compounds specified in claim 1 may also be used.

As used herein, the term "sample" refers to the sample solvent and the inorganic and organic solutes contained within the sample solvent. The sample is typically in the solution phase, but may also exist in other phases of matter including gel, gas-phase, paste or the like. The sample can be altered by changing solvent conditions or by directly changing one or more of the organic agents within the sample. The sample can be altered to create sufficient charge differences between the paramagnetic particle and the target compounds by changing any one of the sample elements or a combination thereof.

For example, but not by way of limitation, changes to the pH or the ionic strength of the sample can associate different charges on the paramagnetic particle and the target compounds. Ionic strength and pH can be optimized to create binding conditions that will differentially bind a target compound mixed with a group of compounds sharing other, less distinguishable physical properties with the target compound. Alternatively, chemistry can be performed on the compounds contained within the sample solvent to promote charge differences between the paramagnetic particle and the target compound. Specific chemistry that can be performed on the target compounds includes, but is not limited to, the esterification of carboxylic groups, the addition of protective groups, or by protein/peptide modification techniques including citraconylation, maleylation, trifluoroacetylation, succinylation, tetrafluorosuccinylation or the like.

If non-specific fractionation through ion exchange is adequate for a particular application, then non-liganded, paramagnetic particles or paramagnetic particles containing only carboxylic or amine functional groups can be utilized.

The present methodology may be used to reduce protein from a sample prior to releasing nucleic acid from a host cell or an infecting organism. This may be helpful in improving nucleic acid binding kinetics. The technique is helpful in instances in which a nucleic acid preparation free of protein is required. In addition, the invention can be used to extract a subset of the total protein sample population by manipulating the protein binding conditions. Using the invention for these purposes gives rise to two distinct uses: (1) selectively binding the protein of interest, discarding the unbound sample or proteins not of interest, and eluting the bound proteins for further analysis (not according to the invention); and (2) where the bound protein does not contain the protein of interest, the bound protein or protein not of interest is discarded and the unbound sample containing the protein of interest is collected for further analysis method according to the invention. Under both scenarios, the compound of interest can be resolved using additional chromatography techniques to further isolate the particular compound of interest from other compounds that share similar charge characteristics.

According to the present disclosure, when a paramagnetic particle carries an electronic charge, the paramagnetic particle will reversibly bind to target molecules having an overall charge opposite that of the paramagnetic particle. The paramagnetic particle and the target molecule, therefore, bond to form a target molecule/particle complex.

Charge may be associated with the paramagnetic particle in any number of ways.

Charge can be associated by attaching charged ligands or functional groups to the paramagnetic particle. Alternatively, charge can be associated to the paramagnetic particle by simply increasing or decreasing the pH of the sample solution surrounding the particle. In either scenariao, the overall charge on the paramagnetic particle can be positive or negative depending on the ligand or functional group (anionic or cationic), pH or ionic strength of the sample.

Although not desiring to be bound by a particular theory, it is believed that when acid is used to associate charge, the acidic enviromnent increases the electropositive nature of the metallic portion of the paramagnetic particle. It is also believed that the low pH conditions increase the binding of the particles to the electronegative portions of a target compound, e.g., in proteins or polypeptides, or regions high in glutamic acid and aspartic acid.

Paramagnetic particles, when placed in a magnetic field, are movable under the action of the field. Such movement is useful for moving bound target compounds in a sample processing protocol or for other manipulations. Thus, target compounds bound to the paramagnetic particles can be immobilized to the interior of a receptacle holding the sample or moved to different areas for exposure to different reagents and/or conditions with minimal direct contact because of the application of magnetic force.

The paramagnetic particles useful in the present invention need not be complicated structures. Suitable paramagnetic particles include iron particles, and the iron may be an iron oxide of forms such as ferric hydroxide and ferrosoferric oxide, which have low solubility in an aqueous environment. Other iron particles such as iron sulfide and iron chloride may also be suitable for binding and extracting target compounds using the conditions described herein.

Similarly, the shape of the paramagnetic particles is not critical to the present invention. The paramagnetic particles may be of various shapes including, for example, spheres, cubes, oval, capsule-shaped, tablet-shaped, nondescript random shapes, etc. and may be of uniform shape or non-uniform shapes. Whatever the shape of the paramagnetic particles, the diameter at the widest point is generally in the range of from about 0.05 µm to about 50 µm, particularly from about 0.1 to about 0.3 µm.

In instances when acid or ionic strength is used to associate charge to the paramagnetic particles or the target compounds, the pH or ionic strength can be provided through a variety of means. For example, the paramagnetic particles can be added to an acidic solution or an acidic solution may be added to the particles. Alternatively, a solution or environment in which the paramagnetic particles are located can be acidified by addition of an acidifying agent such as hydrochloric acid, sulfuric acid, phosphoric acid, acetic acid, citric acid or the like.

Provided that the environment in which the paramagnetic particles are located is of a pH less than about 7.0, the particles will reversibly bind target molecules having an overall negative charge. Furthermore, the protein binding capacity of the paramagnetic particles (without ligands or functional groups attached) increases as the pH decreases. Alternatively, as the solution approaches a neutral or higher pH, and the overall charge on the paramagnetic particles become negative, positively-charged proteins can be bound. As shown below in Example 1, optimal extraction for the paramagnetic particle, ferrosoferric oxide, occurs at pH ranges between 3-4 and 9-10.

Without desiring to be held to a particular theory, it is believed that this technique can replace other crude protein fractionation techniques because the acidic solution of the present invention promotes the binding of electropositive paramagnetic particles to electronegative protein molecules in preference to other substances in a sample such as water-soluble organic salts and other organic reagents.

As stated above, in an acidic environment, electropositive paramagnetic particles, such as ferric oxide particles, will bind electronegative protein molecules. Thus, the present technique can be used to fractionate sample proteins based on charge. Using the protocol of the present technique, one would expect only positively-charged proteins to be extracted. Reagents can be added to samples to impart overall negative charge on sample proteins. For example, lysine residues could be reversibly modified by citraconylation. Likewise, arginine residues could be modified by 1,2-cyclohexanedione. Other means of introducing a negative charge to proteins include maleylation, trifluoroacetylation, succinylation and tetrafluorosuccinylation. Various detergents, such as, e.g., sodium dodecylsulfate (SDS), could also be used.

A similar approach to protein modification can also be used to impart an overall positive charge on proteins, thereby preventing binding. This could be done to improve extraction efficiency and product purity by adding another means to fractionate the protein sample. Materials other than the protein to be bound could, therefore, be positively charged so that they are not attracted to the negatively-charged paramagnetic particles. The positively-charged material would remain in solution so that it could be extracted from the bound protein held by the paramagnetic particles. Such separation can be accomplished by means known to those skilled in the art such as centrifugation, filtering, application of magnetic force and the like.

The bound protein molecules can then be eluted into an appropriate buffer for further manipulation or characterization by various analytical techniques. The elution may be accomplished by heating the environment of the particles with bound proteins and/or raising the pH of the environment. Agents that may be used to aid the elution of protein from paramagnetic particles include basic solutions such as potassium hydroxide, sodium hydroxide or any compound that will increase the pH of the environment to an extent sufficient to displace electronegative protein from the particles.

The present invention provides methods capable of using paramagnetic particles to isolate compounds using affinity-based chromatography. According to these methods, at least one paramagnetic particle is added to a sample receptacle containing one or more organic or biological target compounds. The paramagnetic particle has covalently attached thereto one or more ligands that have an affinity for one or more of the target compounds. The ligands are allowed to interact and contact the target compounds, thereby forming a particle-compound complex. The complex is then immobilized by applying an external magnetic field. The unbound sample or the immobilized complex can then be removed from the sample receptacle. If the immobilized complex is removed from the sample, additional chemistry or chromatography can be applied to the sample. The invention concerns the depletion of high abundance proteins from serum prior to evaluating the sample by an analytical instrument such as a mass spectrometer. Because of the large concentrations of serum albumin, immunoglobulins, and transferrin relative to other serum proteins, removal of these proteins prior to sample analysis by mass spectrometry greatly enhances resolution of other proteins or compounds. Specifically, Protein A and/or Protein G are utilized as ligands to bind immunoglobulins. Native Protein G also has a binding site for albumin. This binding site is usually engineered out so that the Protein G is more specific for immunoglobulins. However, native Protein G with its human albumin binding site, can be utilized in the present invention as a ligand with a paramagnetic particle to demonstrate an affinity chromatography system capable of high throughput albumin depletion for mass spectrometry sample preparation.
The present invention is used to deplete select compounds not of interest from a sample by binding the compounds to one or more solid-phase paramagnetic particle, and subsequently removing the unwanted compound from the sample. For example, when combining serum and paramagnefiic particles containing ligands with a particular affinity for the protein albumin (in the present invention, native Protein G), preferential binding of albumin occurs leaving behind proteins of interest such as disease markers. Using this approach in conjunction with automated equipment (such as the Becton, Dickinson and Company ("BD") Viper platform) equipped with a magnetic extraction block allows easy automation of the fractionation/isolation protocol.

The affinity chromatography method removes the unbound sample from the sample receptacle leaving the immobilized complex behind. The magnetic field can be removed releasing the complex into the receptacle so that additional chemistry can be perfonned on the complex including, but not limited to, releasing the target compound from the paramagnetic particle. In either of the above scenarios, i.e., removing the complex or retaining the complex in the receptacle, the method can be used in conjunction with hardware incorporating external magnets (such as the BD Viper platform) to enable automated high-throughput sample fractionation and compound isolation.

Additional ligand/receptor systems can be used with paramagnetic particles to create other affinity chromatography systems. In addition to Protein A and Protein G, antibodies, antigens, haptens, receptors, enzymes and polypeptide and polynucleotide sequences can all be used as the ligand or functional group with good effect (not according to the invention). In addition, paramagnetic particles have been combined with biological linkages including streptavidin-biotin, avidin-biotin, carbohydrate-lectins, and enzyme-enzyme inhibitor systems (not according to the invention).

The present disclosure also describes kits for isolating proteins from samples, with the kits comprising at least one paramagnetic particle. The kits may also include a source for imparting or altering the charge of the paramagnetic particle, such as an acid. The kit may also include a magnet or another means for creating a magnetic field to be used in the methods described herein. The kits of the current disclosure may or may not include standard labware that may be used in performing the methods of the current invention, such as tubes, syringes, and filter paper.

The following Examples illustrate the present disclosure.

### Example

### Example 1- Magnetic Particle Based Affinity Chromatography To Enhance Mass Spectroscopic Analysis

This Experiment was performed to evaluate magnetic particle-based affinity chromatography as a means to reduce plasma albumin and IgG content, thereby enhancing mass spectroscopic analysis of other sample proteins of interest. The procedure is outlined in Table I below:

**Table I**

| STEP | EVENT |
|---|---|
| 1. | Wash strept-avidin magnetic particles 3X with 1X PBS, place magnet next to tube to immobilize particles, and remove supernatant by aspiration. |
| 2. | Resuspend magnetic particles with 1X PBS to a concentration of 6 mg/mL. |
| 3. | Add 25 µL of 4mg/mL biotinylated Rabbit anti-human serum albumin to tube containing 200 µL of 6 mg/mL washed strept-avidin magnetic particles (Tube 1). |
| 4. | Add 25 µL of 2.9 mg/mL biotinylated monoclonal anti-human IgG1 to tube containing 200 µL of 6 mg/mL washed strept-avidin magnetic particles (Tube 2). |
| 5. | Incubate both tubes 30 minutes with gentle mixing on a rotating stand. |
| 6. | Mix and transfer 100 µL from Tube 1 and 100 µL from Tube 2 to a new tube (Tube 3). |
| | Tube 1: Magnetic Particle - Strept-avidin - Biotin - Rabbit Anti-human Albumin |
| | Tube 2: Magnetic Particle - Strept-avidin - Biotin - Monoclonal Anti-human IgG1 |
| | Tube 3: Equal mix of Tube 1 and Tube 2 particles (Anti- human Albumin and Anti-human IgG1) |
| 7. | Wash tubes 1,2, and 3 three times with 1X PBS, place magnets next to tubes to immobilize particles, and remove supernatant by aspiration. |
| 8. | Dilute 20 µL human plasma sample 1:10 by adding to 180 µL 1X PBS |
| 9. | Transfer 20 µL 1:10 diluted human plasma to each of 3 tubes (Tube 1, 2, and 3) and incubate for 10 minutes |
| 10 | Remove supernatant by aspiration after placing tubes next to magnets to immobilize particles. |
| 11. | Analyze the 3 particle treated samples by mass spectrometry using Ciphergen WCX2 chips. |
| 12. | Dilute untreated plasma 1:30 with 1X PBS and analyze by mass spectrometry (WCX2 chip). This sample serves as a control for Tubes 1, 2, and 3. |
| 13. | Use 50% acetonitrile containing 0.1% TFA as mass spectrometry matrix. |

The procedure used to conduct the experiment, outlined in Table I, begins by washing strept-avidin coated magnetic particles three times with 1X phosphate buffer solution (PBS). The PBS solution is removed by aspiration and the particles are immobilized by placing a magnet next to the collection vessel. In the second step the magnetic particles are resuspended with 1X PBS to a concentration of 6 mg/mL. In step three, 25 µL of 4 mg/mL biotinylated Rabbit anti-human serum albumin is added to the collection vessel (Tube 1) containing 200 µL of 6 mg/mL washed strept-avidin magnetic particles. In step four, a second collection vessel, (Tube 2) is produced by adding 25 µL of 2.9 mg/mL biotinylated monoclonal anti-human IgG1 to a tube containing 200 µL of 6 mg/mL washed strept-avidin magnetic particles. In step five, both collection vessels (Tube 1 and Tube 2) are incubated for 30 minutes with gentle mixing. Tubes 1 and 2 are mixed in step six to generate a third collection vessel, (Tube 3). Tube 3 is an equal mix of Tube 1 and Tube 2. In step seven the three collection vessels are washed with 1X PBS. The PBS is again removed by aspiration following immobilization of the particles by an external magnet. In step eight, 20 µL of human plasma sample is diluted 1:10 by adding 180 µL of 1X PBS. A 20 µL aliquot of the dilute human plasma sample (step eight) is added to each of Tubes 1, 2, and 3. The tubes are incubated for 10 minutes to complete step nine. The supernatant in each of Tubes 1, 2, and 3 is removed in step ten following immobilization of the strept-avidin magnetic particles by an external magnet. In step eleven particles from each tube are analyzed by mass spectrometry using Ciphergen WCX2 chips. Dilute untreated plasma 1:30 with 1X PBS is also analyzed by mass spectrometry (WCX2 chip). This sample serves as a control for the particles analyzed from Tubes 1, 2, and 3. The mass spectrometry matrix used in the control and for all samples is 50% acetonitrile containing 0.1% TFA .

The experimental results of example 2 are graphically displayed in Fig. 1. Fig. 1 compares the mass spec chromatograms of Tubes 1, 2, and 3 against an untreated human plasma sample diluted 1:30 with 1X PBS, which serves as control. In both tube 1 (magnetic particles + anti-human albumin) and tube 2 (magnetic particles + anti-human IgG1), at least six distinct peaks are resolved that are either not detectable in the control sample or barely rise above the noise associated with the chromatogram baseline. The six peaks appear in the form of two relatively small molecular weight proteins (1), (2) between 5 and 10,000 Da and four larger proteins (3), (4), (5) and (6) at approximately 15,000 (peaks (3) and (4)), 20,000 and 26,000 Da. This pattern remains consistent in the chromatogram for tube 3 (magnetic particles + anti-human albumin + anti-human IgG1), which also shows six additional well-resolved peaks.

The resolution of additional protein peaks indicates that treating human plasma samples with magnetic particles containing anti-albumin and/or anti-IgG prior to mass spectrometry, can enhance detection of other proteins of potential interest. Use of these particles on an automated system proven to effectively manipulate magnetic particles and fluids, would enable high throughput automated sample preparation for mass spectrometry.

## Claims

1. A method for depleting high abundance proteins from a serum sample prior to evaluating the sample by an analytical instrument, said sample comprising a target compound and one or more compounds not of interest, the method comprising:
a) adding at least one paramagnetic particle to a sample receptacle containing a serum sample comprising the target compound and compounds not of interest, wherein said paramagnetic particle has one or more ligands covalently attached thereto and said one or more ligands have an affinity for one or more of the compounds not of interest in said sample, wherein said one or more ligands are selected from the group consisting of protein A and native protein G; wherein
when the ligand is native protein G the one or more compounds not of interest are selected from immunoglobulins and albumin, and
when the ligand is protein A the one or more compounds not of interest are selected from immunoglobulins; and wherein
said at least one paramagnetic particle is selected from the group consisting of: a metal selected from the group consisting of iron, cobalt and nickel; and a metallic compound chosen from the group consisting of iron oxide, iron sulfide, iron chloride, ferric hydroxide, and ferrosoferric oxide;
b) contacting the ligands with the one or more compounds not of interest to form a particle-compound complex;
c) immobilizing the complex by applying an external magnetic field; and
d) removing from the receptacle the portion of the sample not immobilized by the magnetic field, wherein the sample thus removed contains the target compound.

2. The method of claim 1 further comprising the step, e) evaluating the removed portion of the sample by mass spectrometry.

## Patentansprüche

1. Verfahren zur Abreicherung reichlich vorhandener Proteine aus einer Serumprobe vor der Bewertung der Probe durch ein analytisches Instrument, wobei die Probe eine Zielverbindung und eine oder mehrere nicht interessierende Verbindungen umfasst, wobei das Verfahren umfasst:
a) Hinzufügen wenigstens eines paramagnetischen Teilchens in einen Probenbehälter, der eine Serumprobe enthält, die die Zielverbindung und die nicht interessierenden Verbindungen umfasst, wobei das paramagnetische Teilchen einen oder mehrere Liganden aufweist, die daran kovalent gebunden sind, und der eine oder die mehreren Liganden eine Affinität zu einer oder mehreren der nicht interessierenden Verbindungen in der Probe aufweisen, wobei der eine oder die mehreren Liganden aus der Gruppe ausgewählt sind, die aus Protein A und nativem Protein G besteht; wobei
wenn es sich bei dem Liganden um natives Protein G handelt, die eine oder die mehreren nicht interessierenden Verbindungen aus Immunglobulinen und Albumin ausgewählt sind; und
wenn es sich bei dem Liganden um Protein A handelt, die eine oder die mehreren nicht interessierenden Verbindungen aus Immunglobulinen ausgewählt sind; und wobei
das wenigstens eine paramagnetische Teilchen aus der Gruppe ausgewählt ist, die aus einem Metall, das aus der Gruppe ausgewählt ist, die aus Eisen, Cobalt und Nickel besteht, und einer Metallverbindung, die aus der Gruppe ausgewählt ist, die aus Eisenoxid, Eisensulfid, Eisenchlorid, Eisen(III)hydroxid und Eisen(II,III)oxid besteht, besteht;
b) In-Kontakt-Bringen der Liganden mit der einen oder den mehreren nicht interessierenden Verbindungen unter Bildung eines Teilchen-Verbindung-Komplexes;
c) Immobilisieren des Komplexes durch Anlegen eines äußeren Magnetfelds; und
d) Entnehmen des Teils der Probe, der nicht durch das Magnetfeld immobilisiert wird, aus dem Behälter, wobei die so entnommene Probe die Zielverbindung enthält.

2. Verfahren gemäß Anspruch 1, das weiterhin den Schritt umfasst:
e) Bewerten des entnommenen Teils der Probe durch Massenspektrometrie.

## Revendications

1. Procédé de déplétion de protéines à abondance élevée d'un échantillon de sérum avant d'évaluer l'échantillon par un instrument analytique, ledit échantillon comprenant un composé cible et un ou plusieurs composé(s) qui n'est/ne sont pas d'intérêt, le procédé comprenant le fait :
a) d'ajouter au moins une particule paramagnétique à un réceptacle d'échantillon contenant un échantillon de sérum comprenant le composé cible et des composés qui ne sont pas d'intérêt, où ladite particule paramagnétique a un ou plusieurs ligand(s) lié(s) de manière covalente à celle-ci et ledit ou lesdits plusieurs ligand(s) a/ont une affinité pour un ou plusieurs des composés qui ne sont pas d'intérêt dans ledit échantillon, où ledit ou lesdits plusieurs ligand(s) est/sont choisi(s) dans le groupe constitué de la protéine A et de la protéine G native ; dans lequel
lorsque le ligand est la protéine G native, le ou les plusieurs composé(s) qui n'est/ne sont pas d'intérêt est/sont choisi(s) parmi les immunoglobulines et l'albumine, et
lorsque le ligand est la protéine A, le ou les plusieurs composé(s) qui n'est/ne sont pas d'intérêt est/sont choisi(s) parmi les immunoglobulines ; et dans lequel
ladite au moins une particule paramagnétique est choisie dans le groupe constitué : d'un métal choisi dans le groupe constitué du fer, du cobalt et du nickel ; et d'un composé métallique choisi dans le groupe constitué de l'oxyde de fer, du sulfure de fer, du chlorure de fer, de l'hydroxyde ferrique et de l'oxyde ferrosoferrique ;
b) de mettre en contact les ligands avec le ou les plusieurs composé(s) qui n'est/ne sont pas d'intérêt pour former un complexe particule-composé ;
c) d'immobiliser le complexe en appliquant un champ magnétique externe ; et
d) de retirer du réceptacle la partie de l'échantillon non immobilisée par le champ magnétique, où l'échantillon ainsi retiré contient le composé cible.

2. Procédé de la revendication 1, comprenant en outre l'étape e) d'évaluation de la partie retirée de l'échantillon par spectrométrie de masse.
